# EUROPEAN PATENT APPLICATION

(11) **EP 1 767 205 A1**
(43) Date of publication of application: **28.03.2007**
(21) Application number: 05758202.5
(22) Date of filing: 05.07.2005
(51) Int. Cl.: A61K 31/353

(54) **LIPASE INHIBITORS**

(30) Priority: 05.07.2004 JP 2004198303
(71) Applicant: SUNTORY LIMITED, Osaka-shi, Osaka 530-8203 (JP)
(72) Inventor: NAKAI, Masaaki, Osaka 5620041 (JP); FUKUI, Yuko, Takatsuki-shi, Osaka 5691123 (JP); ASAMI, Sumio, Ibaraki-shi, Osaka5670886 (JP); HASHIMOTO, Fumio, Kagoshima-shi, Kagoshima 8900081 (JP)
(74) Representative: Vossius & Partner
(86) International application number: PCT/JP2005/012392
(87) International publication number: WO 2006/004109

(57) **Abstract**

The present invention provides lipase inhibitors containing dimers having an epigallocatechin gallate (EGCG) unit derived from teas as well as foods and beverages and medicines containing said inhibitors. More specifically, the present invention provides lipase inhibitors containing at least one of proanthocyanidins represented by the formula: 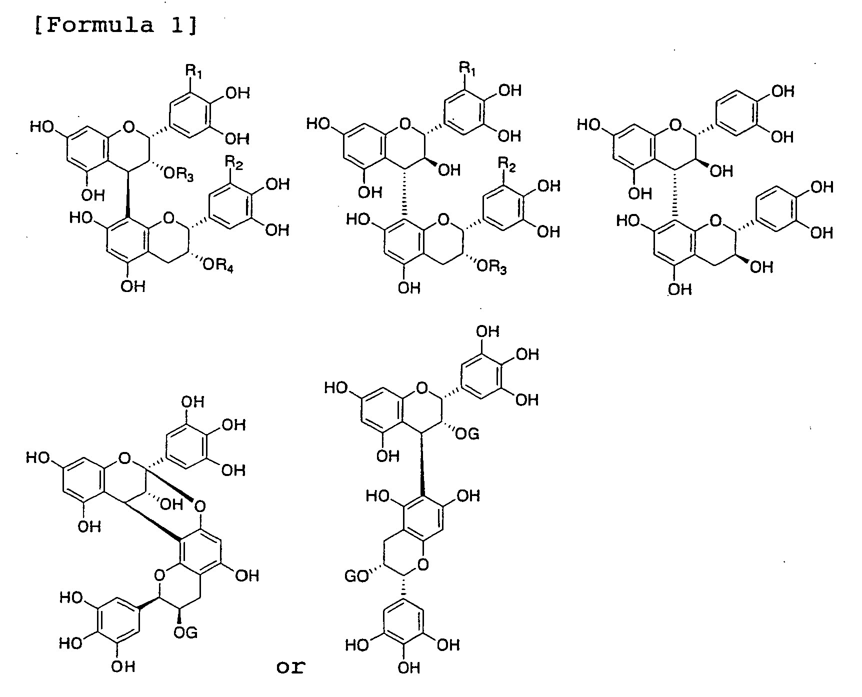 wherein R₁ and R₂ independently represent H or OH, and R₃ and R₄ independently represent H or a galloyl group; as well as foods and beverages and pharmaceutical compositions containing said lipase inhibitors.

## Description

### TECHNICAL FIELD

The present invention provides lipase inhibitors containing proanthocyanidins derived from teas.

### BACKGROUND ART

In recent years, intake of high-fat foods in Japanese people has been increasing with the increasing westernization of their life style. The 1999 National Nutrition Survey reports that the fat energy ratio exceeds the proper level of 25% despite of the energy intake decreasing year by year and that 50 to 60 percent of the 60 and older population has high triglyceride levels or high cholesterol levels (Ministry of Health, Labor and Welfare of Japan. An overview of the results of the 1999 National Nutrition Survey. Japanese Journal of Clinical Nutrition 2001; 98(5): 577-588).

Obesity is one of the most important diseases in modern society, and mainly caused by excessive consumption of fats. Excessive consumption of fats is known to induce not only obesity but also obesity-associated conditions such as diabetes, hyperlipemia, hypertension and arteriosclerosis. An appetite suppressant Mazindol® is the only drug approved for this obesity in Japan, but it was reported to have adverse side effects such as dry mouth, constipation, stomach discomfort and nausea/vomiting (Clinical Evaluation 1985; 13(2): 419-459; Clinical Evaluation 1985; 13(2): 461-515). Outside Japan, a commercially available drug for improving obesity is Xenical®, which functions to suppress intestinal fat absorption by lipase inhibitory activity, but it is not always safe because it was also reported to have adverse side effects such as fatty stools, increased stool frequency, loose stools, diarrhea and abdominal pain (Lancet 1998; 352: 67-172).

An effective means to prevent obesity is to reduce caloric intake by dietary restrictions, but they should be supervised by an experienced nutrition counselor and it is often difficult to follow them in daily life. Thus, a safe and healthful way to inhibit the absorption of dietary fats by the body would be a practical and useful approach to the treatment of obesity and related diseases or health enhancement.

Against this background, attention has been given to the development of foods for specified health use with proven safety and effectiveness for humans. Foods for specified health use so far marketed as food materials for controlling the increase in serum triglyceride levels after eating include globin digests suppressing fat absorption by pancreatic lipase inhibition (J. Nutr. 1998; 128: 56-60; Journal of the Japanese Society of Nutrition and Food Science 1999; 52(2): 71-77; Journal of Health Food & Nutrition Food Studies 2002; 5(3): 131-144); diacylglycerols having different digestion/absorption characteristics from those of triacylglycerols (J. Am. Coll. Nutr. 2000; 19(6): 789-796; Clin. Chim. Acta. 2001; 11(2): 109-117); and eicosapentaenoic acid (EPA) and docosahexaenoic acid (DHA) purified from fish oils, etc.

Attention is also recently being given to plant-derived materials having lipase inhibitory activity, and especially various polyphenols having lipase inhibitory activity have been reported, such as plant bark-derived tannin (Japanese Patent Publication Sho 60-11912); tannins and flavonoids and glycosides thereof contained in a legume *Cassia* nomame (Japanese Patent Laying Open Hei 8-259557); food products for inhibiting lipid absorption containing epigallocatechin gallate and epicatechin gallate known as major ingredients in green tea (Japanese Patent Laying Open Hei 3-228664); lipase inhibitors comprising aqueous extracts of green pepper, shimeji mushroom, pumpkin, maitake mushroom, hijiki seaweed, green tea, oolong tea, etc. (Japanese Patent Laying Open Hei 3-219872); flavones and flavonols (Japanese Patent Laying Open Hei 7-61927); hydroxybenzoic acids (gallic acid) (Japanese Patent Laying Open Hei 1-102022); triterpene compounds and derivatives thereof (Japanese Patent Laying Open Hei 9-40689); anti-obesity agents containing procyanidin from tamarind as an active ingredient (Japanese Patent Laying Open Hei 9-2-91039); as well as lipase inhibitory effects of grape seed extracts (Nutrition 2003; 19(10): 876-879); lipase inhibitory effects and anti-obesity effects in rats of Salacia-derived polyphenols (J. Nutr. 2002; 132: 1819-1824); and anti-obesity effects of oolong tea extracts in mice (Int. J. Obes. 1999; 23: 98-105).

However, plant-derived lipase inhibitors so far reported as shown above are not sufficiently effective. Even if an extract of a plant was effective, for example, it would be difficult to stably maintain lipase inhibitory activity unless the amount of the active ingredient contained in it is specified because it is naturally derived. Moreover, inhibitors derived from tasteless plants have the disadvantage that they affect flavor when they are used as foods or beverages. For example, there are several reports showing the effect of oolong tea in improving lipid profiles by demonstrating a significant decrease in blood triglyceride levels after drinking 1330 ml of commercially available oolong tea daily for 6 weeks (Journal of the Japanese Society of Nutrition and Food Science 1991; 44(4): 251-259) or a weight loss of 1 kg or more in 67% of subjects consisting of 102 men and women with simple obesity who continuously took oolong tea (2 g x 4/day) orally for 6 weeks and a significant improving effect after ingestion of oolong tea in subjects showing high blood triglyceride levels (Journal of the Japanese Society of Clinical Nutrition 1998; 20(1): 83-90). Thus, beneficial effects have been observed by drinking plenty of oolong tea, but it is difficult to continue to do so in daily life. If simply concentrated oolong tea was provided, it would not be suitable as a practical means because of strong bitterness/astringency and high caffeine content.

### Patent documents

1. Japanese Patent Publication Sho 60-11912
2. Japanese Patent Laying Open Hei 8-259557
3. Japanese Patent Laying Open Hei 3-228664
4. Japanese Patent Laying Open Hei 3-219872
5. Japanese Patent Laying Open Hei 7-61927
6. Japanese Patent Laying Open Hei 1-102022
7. Japanese Patent Laying Open Hei 9-40689
8. Japanese Patent Laying Open Hei 9-291039

### Non-patent documents

1. Ministry of Health, Labor and Welfare of Japan. An overview of the results of the 1999 National Nutrition Survey.
2. Japanese Journal of Clinical Nutrition 2001; 98(5): 577-588.
3. Clinical Evaluation 1985; 13(2): 419-459. Clinical Evaluation 1985; 13(2): 461-515.
4. Lancet 1998; 352: 67-172.
5. J. Nutr. 1998; 128: 56-60.
6. Journal of the Japanese Society of Nutrition and Food Science 1999; 52(2): 71-77.
7. Journal of Health Food & Nutrition Food Studies 2002; 5(3): 131-144.
8. J. Am. Coll. Nutr. 2000; 19(6): 789-796.
9. Clin. Chim. Acta. 2001; 11(2): 109-117.
10. Nutrition 2003; 19(10): 876-879.
11. J. Nutr. 2002; 132: 1819-1824.
12. Int. J. Obes. 1999; 23: 98-105.
13. Journal of the Japanese Society of Nutrition and Food Science 1991; 44(4): 251-259.
14. Journal of the Japanese Society of Clinical Nutrition 1998; 20(1): 83-90.
15. Chem. Pharm. Bull 1983; 31(11): 3906-3914.
16. Chem. Pharm. Bull 1989; 37(1): 77-85.

### DISCLOSURE OF THE INVENTION

### PROBLEM TO BE SOLVED BY THE INVENTION

The present invention focuses on ingredients contained in highly tasty teas and provides lipase inhibitors containing at least one of proanthocyanidins derived from teas.

The present invention also provides highly tasty foods and beverages containing said lipase inhibitors for reducing blood triglycerides and for enhancing health.

The present invention also provides pharmaceutical compositions containing said lipase inhibitors for inhibiting absorption of dietary fats to prevent an increase in blood triglycerides.

### MEANS FOR SOLVING PROBLEM

As a means for solving the above problems, we found tea-derived ingredients inhibiting pancreatic lipase essential for fat absorption, and evaluated the lipase inhibitory activity of various polyphenols present therein, and ascertained that proanthocyanidins, especially proanthocyanidins having a gallate group have strong lipase inhibitory activity.

More specifically, lipase inhibitors of the present invention are characterized in that they contain at least one of proanthocyanidins represented by the formula:

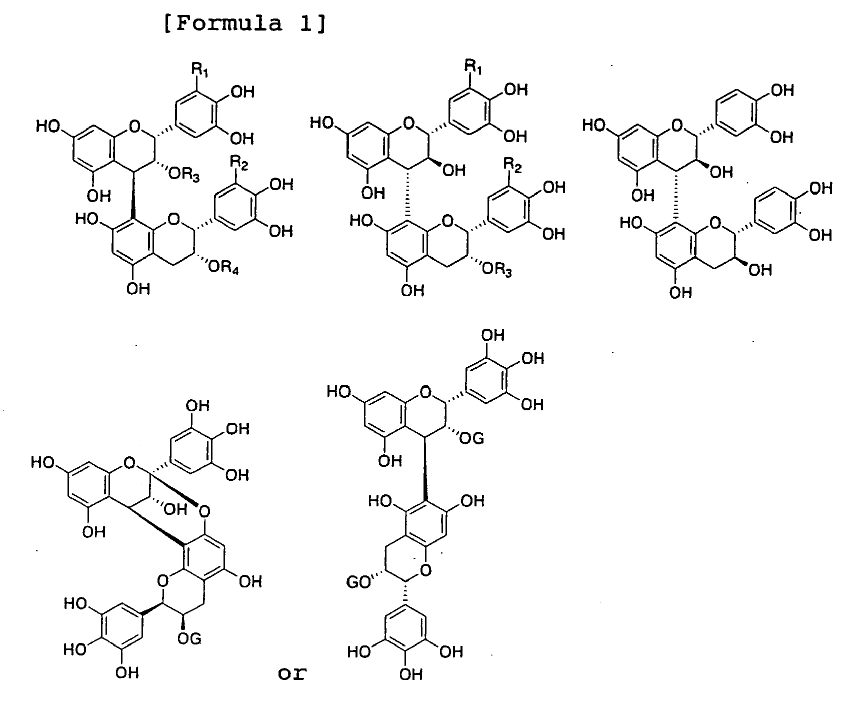

wherein R₁ and R₂ independently represent H or OH, and R₃ and R₄ independently represent H or a galloyl group.

The galloyl group G has the structural formula:

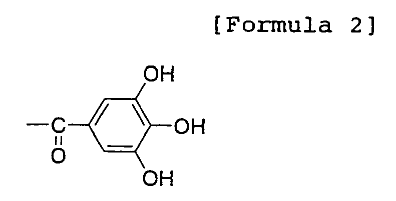

Proanthocyanidins of the present invention can be extracted/purified from natural materials such as commercially available green tea, black tea and oolong tea by the process described in Chem. Pharm. Bull. 1983; 31(11): 3906-3914, 1983 or Chem. Pharm. Bull. 1989; 37(1): 77-85, for example.

### Lipase inhibitors

Proanthocyanidins of the present invention can be used alone as lipase inhibitors without including other components, or can be used as lipase inhibitors in combination with solvents or solid carriers. The solvents or carriers are preferably those capable of being safely used as foods or medicines in terms of the uses for foods and beverages and/or medicines as described below. Lipase inhibitors of the present invention have various uses such as experimental and research purposes or uses as active ingredients of foods and medicines for preventing accumulation of triglycerides.

### Method for assaying lipase inhibitory activity

Lipase inhibitors of the present invention have a strong inhibitory effect against lipases, especially pancreatic lipase. The inhibitory activity can be assayed by the method specifically described in Example 1.

### Foods and beverages containing lipase inhibitors

Lipase inhibitors containing proanthocyanidins of the present invention can be added as active ingredients for inhibiting lipase to foods and beverages to prevent an undesirable increase in blood triglycerides associated with intake of dietary fats and/or reduce increased blood triglycerides. Preferred examples of foods and beverages include those consumed on a daily basis such as green tea, barley tea, oolong tea, black tea, coffee, isotonic drink, drinking water, seasonings, and dressings. However, the foods and beverages may be those commonly consumed such as soft drinks, cocktails, beer, whiskey, distilled spirits, wine, sake, seasonings, dressings, flavored rice, processed foods, convenience foods, retort foods, chocolates, fresh cream, cakes, dairy products, health foods and supplements.

Lipase inhibitors of the present invention are added to foods and beverages in an amount corresponding to an intake of proanthocyanidins of 0.1 mg to 10 g per meal. However, there is no substantial upper limit on the amount of proanthocyanidins of the present invention that can be added to foods and beverages because they are derived from foods and therefore very safe.

### Medicines containing lipase inhibitors

Lipase inhibitors containing proanthocyanidins of the present invention can also be used as active ingredients of drugs for inhibiting absorption of dietary fats and preventing and/or reducing an undesirable increase of blood triglycerides. Preferred drugs are those orally administered, such as drinkable preparations, tablets, capsules, granules, powders, candies and hard candies. The amount of compounds of the present invention is 0.1 mg to 10 g per dose.

Medicines of the present invention are safely taken even for a long period because of high safety of lipase inhibitor ingredients. Therefore, they can be taken even on a daily basis to prevent or correct obesity as a lifestyle-related disease.

### EFFECT OF THE INVENTION

The present invention can provide highly tasty foods and beverages containing a lipase inhibitor including at least one of proanthocyanidins derived from tea leaves for reducing triglycerides and for enhancing health without compromising flavor. Beverages enriched with tea-derived active ingredients are very significant because the inhibitor should desirably be taken with meals in order to inhibit absorption of dietary fats. Especially, the present invention made it possible to develop teas capable of reducing triglycerides by enriching them with these ingredients.

### BRIEF EXPLANATION OF THE DRAWINGS

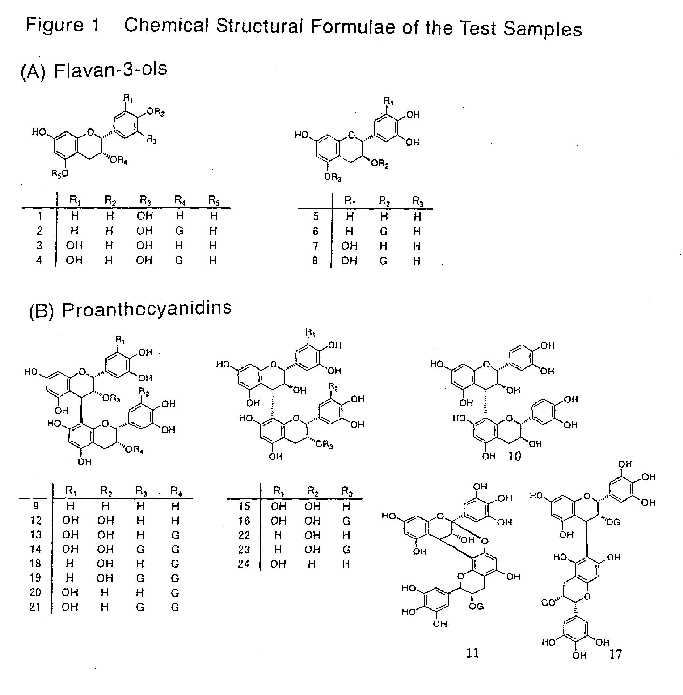

Figure 1 shows the chemical structural formulae of the compounds evaluated for lipase inhibitory activity in Example 2.

### EXAMPLES

### Examples 1: Lipase inhibitory activity assay

A lipase activity assay was performed by using the oleate ester of fluorescent 4-methylumbelliferone (4-MUO) as a substrate to measure the fluorescence of 4-methylumbelliferone produced by reaction.

The buffer used for the assay was 13 mM Tris-HCl (pH 8.0) containing 150 mM NaCl, 1.36 mM CaCl₂. The enzyme assay was performed using a 0.1 M solution of the substrate 4-MUO (Sigma) in DMSO diluted 1:1000 in said buffer and a solution of porcine pancreatic lipase (Sigma) prepared at 400 U/ml also in said buffer.

An enzymatic reaction was started by adding 25 µl of the lipase/buffer solution after 50 µl of the 4-MUO/buffer solution and 25 µl of distilled water (or an aqueous solution of each sample) were added and mixed in a 96-well microplate at 25°C. After the reaction was performed for 30 minutes, the reaction was stopped by adding 100 µl of a 0.1 M citrate buffer (pH 4.2) and the fluorescence of 4-methylumbelliferone produced by the reaction (excitation 355 nm, emission 460 nm) was measured using a fluorescence plate reader (Fluoroskan Asent CF from Labsystems).

The inhibitory activity of each test sample was determined as IC₅₀(µM), i.e. the amount of the sample giving 50% inhibition of the activity of the control (distilled water).

### Test samples

Control compounds catechin (5), epicatechin (1), gallocatechin (7), epigallocatechin (3), catechin gallate (6), epicatechin gallate (2), gallocatechin gallate (8), and epigallocatechin gallate (4) were purchased from Wako Pure Chemical Industries, Ltd.

Proanthocyanidins of the present invention were obtained by the process described in the following articles: Chem. Pharm. Bull. 1983; 31(11): 3906-3914; Chem. Pharm. Bull. 1989; 37(1): 77-85. Briefly, oolong tea leaves were extracted with 80% acetone, after which acetone was removed and the extract was solvent-fractionated with ethyl acetate. The ethyl acetate layer was fractionated with water, methanol, 50% acetone on Sephadex LH-20 (Pharmacia), and then each fraction was adsorbed to Diaion HP-20 (Mitsubishi Kasei Corp.) and eluted with a water-methanol system, and purified again on Sephadex LH-20 (Pharmacia).

### Example 2: Lipase inhibitory activity of catechins and dimers thereof (proanthocyanidins)

Catechins (monomers) and proanthocyanidins produced by polymerization of catechins known as major polyphenols in teas were assayed for lipase inhibitory activity according to the method of Example 1. The results are shown in Table 1. The chemical structural formulae of the compounds subjected to evaluation are shown in Figure 1. Among major catechins (8 catechins) present in teas, flavan-3-ols having a gallate moiety bonded via an ester linkage showed lipase inhibitory activity. Especially, epigallocatechin gallate (EGCG) abundantly found in teas showed the strongest activity among the major catechins.

Among proanthocyanidins, those having a gallate group in their structure, especially dimers containing an EGCG unit were shown to have higher lipase inhibitory activity than EGCG per se.

[Table 1]

**Table 1. Lipase inhibitory activity of tea-derived polyphenols**

| Polyphenols | IC₅₀ (µM) |
|---|---|
| Flavan-3-ols | |
| (-)-epicatechin (1) | >21.6 |
| (-)-epicatechin 3-O-gallate (2) | 0.271 |
| (-)-epigallocatechin (3) | >20.4 |
| (-)-epigallocatechin 3-O-gallate (4) | 0.284 |
| (+)-catechin (5) | >690 |
| (-)-catechin 3-O-gallate (6) | 0.846 |
| (+)-gallocatechin (7) | >163 |
| (-)-gallocatechin 3-O-gallate (8) | 0.349 |

| Procyanidins | |
|---|---|
| procyanidin B-2 (9) | 7.958 |
| procyanidin B-3 (10) | 2.941 |
| prodelphinidin A-2 3'-O-gallate (11) | 0.171 |
| prodelphinidin B-2 (12) | 2.951 |
| prodelphinidin B-2 3'-O-gallate (13) | 1.969 |
| prodelphinidin B-2 3,3'-di-O-gallate (14) | 0.107 |
| prodelphinidin B-4 (15) | 6.230 |
| prodelphinidin B-4 3'-O-gallate (16) | 0.223 |
| prodelphinidin B-5 3,3'-di-O-gallate (17) | 0.558 |
| (-)-epicatechin (4β-8) (-)-epigallocatechin 3-O-gallate (18) | 0.147 |
| (-)-epicatechin 3-O-gallate (4β-8) (-)-epigallocatechin 3-O-gallate (19) | 0.846 |
| (-)-epigallocatechin (4β-8) (-)-epicatechin 3-O-gallate (20) | 0.913 |
| (-)-epigallocatechin 3-O-gallate (4β-8) (-)-epicatechin 3-O-gallate (21) | 0.612 |
| (+)-catechin (4α-8) (-)-epigallocatechin (22) | 7.912 |
| (+)-catechin (4α-8) (-)-epigallocatechin 3-O-gallate (23) | 0.174 |
| (+)-gallocatechin (4α-8) (-)-epicatechin (24) | 2.862 |

It was shown from the above results that dimers having very high activity also exist in teas in addition to the major catechins (8 catechins).

## Claims

1. A lipase inhibitor containing at least one of proanthocyanidins represented by the formula: 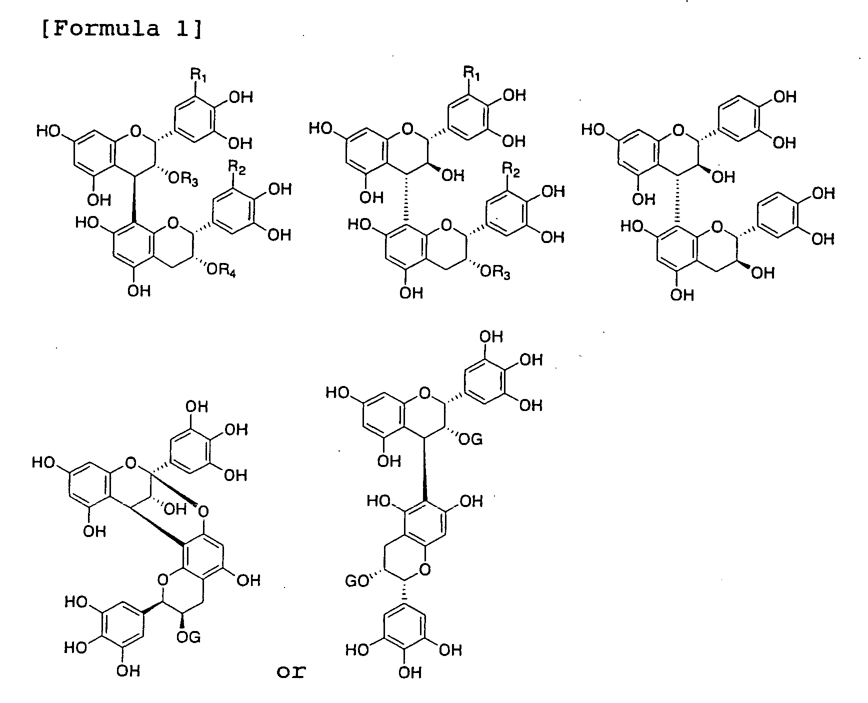 wherein R₁ and R₂ independently represents H or OH, and R₃ and R₄ independently represent H or a galloyl group.

2. The lipase inhibitor of claim 1 wherein the proanthocyanidin is selected from the group consisting of: procyanidin B-2,
procyanidin B-3,
prodelphinidin A-2 3'-O-gallate,
prodelphinidin B-2,
prodelphinidin B-2 3'-O-gallate,
prodelphinidin B-2 3,3'-di-O-gallate,
prodelphinidin B-4,
prodelphinidin B-4 3'-O-gallate,
prodelphinidin B-5 3,3'-di-O-gallate,
(-)-epicatechin(4β-8)(-)-epigallocatechin 3-O-gallate,
(-)-epicatechin 3-O-gallate(4β-8)(-)-epigallocatechin 3-O-gallate,
(-)-epigallocatechin(4β-8)(-)-epicatechin 3-O-gallate,
(-)-epigallocatechin 3-O-gallate(4β-8)(-)-epicatechin 3-O-gallate,
(+)-catechin(4α-8)(-)-epigallocatechin,
(+)-catechin(4α-8)(-)-epigallocatechin 3-O-gallate, and
(+)-gallocatechin(4α-8)(-)-epicatechin.

3. A food or beverage containing the lipase inhibitor of claim 1 or 2.

4. The food or beverage of claim 3, which is selected from the group consisting of tea drinks, soft drinks and health foods.

5. A pharmaceutical composition containing the lipase inhibitor of claim 1 or 2.

6. The pharmaceutical composition of claim 5 for inhibiting absorption of dietary fats.
